(19) Europäisches Patentamt
European Patent Office
Office européen des brevets

(11) Publication number: 0 060 580 B1

# (12) EUROPEAN PATENT SPECIFICATION

(45) Date of publication of patent specification: 05.06.85

(51) Int. Cl.[4]: C 07 B 57/00, C 07 C 121/75, A 01 N 37/38

(21) Application number: 82200227.5

(22) Date of filing: 24.02.82

## (54) Crystalline phenylacetate enantiomer pair, and preparation of a pesticidal enantiomer pair.

(30) Priority: 16.03.81 US 244372

(43) Date of publication of application:
22.09.82 *Bulletin 82/38*

(45) Publication of the grant of the patent:
05.06.85 Bulletin 85/23

(84) Designated Contracting States:
AT BE CH DE FR GB IT LI LU NL SE

(56) References cited:
EP-A-0 040 991
GB-A-2 013 206
GB-A-2 041 366

(73) Proprietor: SHELL INTERNATIONALE RESEARCH MAATSCHAPPIJ B.V.
Carel van Bylandtlaan 30
NL-2596 HR Den Haag (NL)

(72) Inventor: Petty, Walter Leonard
13627 Balmore Circle
Houston Texas 77069 (US)

(74) Representative: Hunter, Keith Roger Ian et al
4 York Road
London SE1 7NA (GB)

EP 0 060 580 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of a pesticidal phenylacetate rich in one pair of enantiomers and to a novel intermediate which is a different enantiomer pair whose crystalline properties are important to the operability of the process.

Specifically, it relates to a method for the preparation of a cyanobenzyl phenylacetate of the formula I

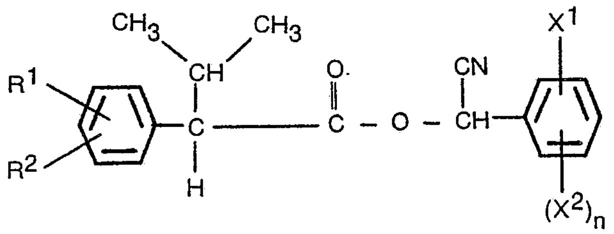

(I)

wherein $R^1$ is a hydrogen atom, a halogen atom, or an alkyl group containing from 1 to 4 carbon atoms or a methoxy or ethoxy group, each optionally substituted by one or more halogen atoms; $R^2$ is a hydrogen atom or a methyl group; $X^1$ is phenoxy, benzyl or phenylthio; $X^2$ is a halogen atom, or is methyl and n is 0, 1 or 2; which is rich in S-alpha-cyanobenzyl S-alpha-isopropylphenyl-acetate and its enantiomer R-alpha-cyanobenzyl R-alpha-isopropyl-phenylacetate (hereinafter referred to as enantiomer pair Y). It also relates to a different pair of enantiomers of a cyanobenzyl phenylacetate of the formula I as defined above. US patent specification No. 4238406 describes a process of preparing enantiomer pair Y of alpha-cyano-3-phenoxybenzyl alpha-isopropyl-4-chlorophenylacetate (fenvalerate) by crystallization from the corresponding racemate, in the presence of seed crystals of enantiomer pair Y. Mother liquors enriched in the S-alpha-cyanobenzyl R-alpha-isopropylphenylacetate and its enantiomer R-alpha-cyanobenzyl S-alpha-isopropylphenylacetate (hereinafter referred to as enantiomer pair X) are not crystallized, but instead are epimerized to form an essentially racemic recycle stream. It follows from US—4238406 that enantiomer pair X of fenvalerate has not been crystallized or even substantially isolated. The process described has the disadvantage that crystals of enantiomer pair Y or Y-enriched crystals are but slowly obtained, especially once—as the Applicant has found—initial enantiomer pair X or X-enriched crystals have been formed.

It is furthermore described in non-prepublished Japanese patent application No. 71940/80, from which a substantial part of European patent application No. 40991 (designating Switzerland, W. Germany, France, Great Britain, Italy and The Netherlands) claims its priority, and which thus is part of the state of the art in the designated countries, how to improve the above-described process. It is proposed therein to crystallize the X-enantiomer pair, and to obtain a mother liquor rich in the Y-isomer, which is the subject of the present invention as well. The application of earlier date does not mention how to prevent epimerization of the enantiomer pair Y contained in the mother liquor, which epimerization may occur especially in the presence of a base, as preferred, affecting the purity of the desired product.

Accordingly the invention is characterized by precipitating crystals enriched in S-alpha-cyanobenzyl R-alpha-isopropylphenylacetate and its enantiomer (hereinafter referred to as enantiomer pair X) in the presence of crystals of enantiomer pair X from a solution of the corresponding racemic R,S-alpha-cyanobenzyl R,S-alpha-isopropylphenylacetate, separating these X-enriched crystals from the mother liquor, redissolving the crystals thus separated in a solvent and treating the resulting solution with a base to epimerize the dissolved enantiomer pair X to the corresponding racemic mixture, recycling the racemic mixture back to the precipitation step and recovering mother liquor enriched in enantiomer pair Y, and (in some countries) further characterized by adding an acid stabiliser to the racemic phenylacetate mother liquor to prevent epimerization of the enantiomer pair Y contained therein.

The present process is quite different from—virtually the converse of—that derscribed in the above U.S. patent. The present process avoids the problem of obtaining crystals of enantiomer pair Y by eliminating the need to obtain crystals thereof, and takes advantage of the previously not published and different properties of crystalline enantiomer pair X and X-enriched crystals. Moreover the very much faster crystallization of enantiomer pair X or X-enriched crystals allows the use of a multistage, continuous crystallizer, if desired, in place of very large batch crystallizers. Continuous processing is often preferred over batch processing for both technical and economic reasons.

The crystalline enantiomer pair X of the invention can be prepared from the corresponding racemic R,S-alpha-cyanobenzyl R,S-alpha-isopropylphenylacetate by passing the racemate through a chromatographic column containing a suitable packing material, for example, silica gel, using an eluant liquid, for example, 1% diethyl ether in hexane. The enantiomer pair X is recovered as the earlier emerging of the two enantiomer pairs. Crystalline enantiomer X can then be obtained by cooling the enantiomer pair X resulting from the chromatographic separation. The cooling (crystallizaton) is conducted at any temperature at which crystals of X form, for example, at about −50° to about 20°C and, preferably about −15° to about 5°C. The crystalline enantiomer pair X of most commercial relevance contains S-alpha-cyano-3-phenoxybenzyl R-alpha-isopropyl-p-chlorophenylacetate and R-alpha-cyano-3-phenoxybenzyl S-alpha-isopropyl-p-chlorophenylacetate. This material is usually recovered in purity greater than 60%, generally about 64%; further recrystallizations yield a product of purity of at least 75% and usually above at least 80% or even above 90%. Crystals enriched in the enantiomer pair X (i.e. containing crystals of enantiomer pair X and other crystalline forms of either of the X diastereoisomers) are also formed during in situ cooling the above described racemate in a solvent, for example, methanol, from which the desired X-enriched crystals spontaneously crystallize preferentially without the addition of X seed crystals, for example, as described below in Example I(A). Repeated recrystallization of the crude crystals, as in Example I(B) below, yields enantiomer pair X in a crystalline form of high purity and melting point of 68°C.

Crystals of enantiomer pair Y have a melting point range and appear to be mixtures of crystals of each of the two isomers which comprise this enantiomer pair. The crystals of enantiomer pair X have unique properties markedly different from those of crystalline pair Y. These unique and different properties include a higher and sharper melting point than crystalline pair Y, which indicates that crystalline enantiomer pair X is a crystalline racemic compound of the two enantiomers which comprise pair X rather than a mixture of crystals of each of the two enantiomers as in crystalline pair Y. In addition, it has been discovered that the solubility of crystalline Y is from 2 to 4 times greater than that of crystalline enantiomer pair X or of crystals enriched in pair X and that enantiomer pair X has a very much faster rate of crystallization than enantiomer pair Y. These properties of crystalline enantiomer pair X and crystals enriched in pair X enable a product enriched in enantiomer pair Y to be prepared by the process of this invention.

The process of the invention is of particular commerical value in the preparation of those cyanobenzyl phenylacetates of formula I wherein $R^1$ is a halogen atom or an optionally halogenated alkyl or alkoxy group, for example a chlorine or fluorine atom, or a methyl, ethyl, isopropyl, tert-butyl, methoxy, ethoxy, difluoromethoxy or trifluoromethoxy group; $R^2$ is a hydrogen atom; $X^1$ is a 3-phenoxy substituent and n is 0. $R^1$ is preferably located at the meta- or, more especially the para-, position relative to the benzyl carbon atom in the acid moiety.

Because of their pesticidal properties, the present process is of particular value in the preparation of a Y-enriched form of a phenylacetate of formula I in which $R^1$ is chlorine or difluoromethoxy, $R^2$ is a hydrogen atom, $X^1$ is 3-phenoxy and n is 0; and especially the compound alpha-cyano-3-phenoxybenzyl alpha-isopropyl-p-chlorophenylacetate.

The solvent used in the process can be any inert liquid material in which the phenylacetate is at least partly soluble at room temperature. Examples of suitable classes of solvents include chlorinated hydrocarbons, ethers, nitriles, esters, amides, hydroxylic solvents and the like. Suitable hydroxylic solvents include lower alkanols containing from 1 to 4 carbon atoms such as isopropanol, butanol, ethanol, and methanol, and preferably containing from 1 to 2 carbon atoms, especially methanol. Other suitable solvents are alkanes containing from 5 to 10 carbon atoms such as n-pentane, n-hexane, n-heptane, n-octane, n-nonane, n-decane and their isomers. Petroleum fractions rich in alkanes are also suitable, for example, gasoline with a boiling range at atmospheric pressure of between 40 to 65°C, between 60 to 80°C or between 80 to 110°C. Petroleum ether is also suitable. Cyclohexane and methylcyclohexanes are examples of useful cycloalkanes containing from 6 to 8 carbon atoms. Aromatic hydrocarbon solvents can contain from 6 to 10 carbon atoms, for example, benzene, toluene, o-, m- and p-xylene, the trimethylbenzenes, p-ethyltoluene and the like. Suitable chlorinated hydrocarbons contain from 1 to 4 chlorine atoms in combination with an alkane chain containing from 1 to 4 carbon atoms or with a benzene ring, for example, carbon tetrachloride, chloroform, dichloromethane, 1,2-dichloroethane, trichloroethane, perchloroethane, chlorobenzene and 1,2- or 1,3-dichlorobenzene. Ethers may be those containing from 4 to 6 carbon atoms such as diethyl ether, methyl tert-butyl ether and diisopropyl ether. Tetrahydrofuran and dioxane are also useful. Suitable nitriles usually also contain from 2 to 6 carbon atoms, for example, acetonitrile and the like. Esters may be those of lower alcohols and acids each containing from 2 to 6 carbon atoms, for example, ethyl acetate. Amides may be those of lower alkyl amines and acids each containing from 1 to 6 carbon atoms, for example, dimethylformamide.

While different solvents may be employed in the crystallization and epimerization steps, it is often convenient to use the same solvent in both steps, with alkanes and alkanols being preferred. Alkanols containing 1 to 4 carbon atoms are particularly useful, especially methanol.

The epimerization catalyst can be any basic agent, e.g., inorganic or organic in nature, which does not itself form stable reaction products with the cyanohydrin ester, and preferably has a $pK_b$ of less than 6. Examples of suitable inorganic compounds include hydroxides, carbonates, hydrides, and cyanides of alkali and alkaline earth metals, and metal oxides such as sodium cyanide, sodium hydroxide, barium hydroxide, potassium hydroxide, calcium carbonate, sodium carbonate, calcium oxide, alumina, zinc oxide and the like.

Suitable organic bases are alkali or alkaline earth metal salts of weak organic acids or organic nitrogen bases, alkali metal alcoholates and alkali metal amides. Suitable salts include sodium acetate, magnesium formate, potassium tert-butylate, sodium isopropylate and the like. Nitrogen bases can be ammonia, ammonium hydroxide or any alkyl, aryl or heterocyclic nitrogen base including mono- or polyamines and the like. Preferably, the organic nitrogen base is an amine in which any alkyl groups contain from 1 to 10 carbon atoms, any aryl or aralkyl groups contain from 6 to 20 carbon atoms and 1 to 2 hydrocarbyl rings, and any heterocyclic amines contain at least one ring nitrogen atom in a 5 or 6 membered heterocyclic ring optionally containing a sulfur or oxygen atom or another nitrogen atom, such as trimethylamine,

diethylamine, triethylamine, piperidine, isoamylamine, benzylamine, l-naphthylamine, diethylamine, tri-n-propylamine, ephedrine, tert-butylamine, ethanolamine, triethylenediamine, tetramethylenediamine, pyrrolidine, quinoline, pyridine, morpholine or tetrabutyl-p-ammonium hydroxide. The amines are preferably secondary and especially tertiary amines containing any combination of the above-described groups. When the amine is a tertiary amine it desirably contains three alkyl groups of 1 to 4 carbon atoms, for example: trimethylamine, tri-n-propylamine, and especially triethylamine.

Other suitable basic agents are ion exchange resins having a strong basic character. Such resins include quaternary ammonium and amine ion exchange resins. Resins of this type are often sold under the trade names Dowex and Amberlite, for example those derived from trimethylamine (such as the products known under the trade names of "Amberlite IRA—400", "Amberlite IRA—40L", "Amberlite IRS—402", "Amberlite IRA—900", "Duolite A—101—D", "Duolite ES—111", "Dowex 1", "Dowex 11", "Dowex 21K" and "Ionac A—450"), and those derived from dimethylethanolamine (such as the products known under the trade names of "Amberlite IRA—410", "Amberlite IRA—911", "Dowex 2", "Duolite A—102—D", "Ionac A—542" and "Ionax A—550"). Very good results have been obtained with those derived from trimethylamine. When these catalysts are available in a neutralized form, for instance in the chloride form, they must be activated to the hydroxyl form by treatment with an aqueous alkali metal hydroxide, for example sodium hydroxide, and washed with water to remove salt anions before use. Also useful as basic agents are high molecular weight liquid amines which are insoluble in water such as the "liquid Amberlites" which are sold commercially as liquid Amberlites of the type LA1 and LA2.

Phosphorus-containing bases are also suitable, such as lower alkyl phosphines like triphenyl phosphine and tri-n-butyl phosphine.

The preferred epimerisation base is ammonia or a tertiary alkyl amine wherein each alkyl group contains from 1 to 4 carbon atoms, such as triethylamine.

The concentration of the epimerization catalyst may vary from 0.001 to 100 mole % and suitably from 0.01 to 50 mole % based on the amount of racemate, preferably from 0.05 to 20 mole %, and especially from 0.1 to 15 mole %. Normally about 1 mole % is used.

A stabilizing amount of an acid may be, or is, added to the racemic mixture solution prior to crystallization in order to neutralize catalyst or other basic materials and prevent epimerization of the enantiomer pair Y in the mother liquor during subsequent crystallization of crystals enriched in enantiomer pair X.

While the precise amount of acid needed will depend upon the amount of basic catalyst used or other basic material present, from 0.001 to 5%, preferably from 0.01 to 0.5%, by weight of acid based upon the phenylacetate feed may be used.

Any inorganic or organic acid or acidic acting material which will not undesirably affect the desired product can be used to stabilize the solution, for example, mineral acids, such as hydrochloric or sulphuric acid, sulphonic acids, such as toluenesulphonic acid, or organic acids, including lower alkanoic acids, such as acetic, propionic or butyric acid. Acetic acid is preferred.

Precipitation, e.g. crystallization, is conducted by forming a mixture of the racemate in a suitable solvent as defined above. The process can be conducted at any temperature at which crystals enriched in enantiomer pair X form, suitably from −50° to 60°C, preferably from −35° to 5°C and especially from −15° to 5°C.

It is often desirable to add substantial amounts of seed crystals to enhance the rate of crystallization. It is usually most convenient to use crystals of the essentially pure enantiomer pair X or crystals enriched therein as seed crystals, although crystals of either single diastereoisomer in enantiomer pair X or a mixture of crystals of both diastereoisomers in enantiomer pair X can be used. Use of high purity enantiomer pair X seed crystals appears to lead to higher yields of enantiomer pair Y in the filtrate. Other known nucleating agents can be used when seed crystals are desired, for example, powdered silica, potassium acetate and the like. The amount of seed crystals used is not critical, but the crystallization is faster with a large amount of seed crystals. The amount of seed crystals may vary from 0.05 to 10% based upon the phenylacetate in solution and is preferably about 1%. Of course, as the process progresses, the crystals of enantiomer pair X being formed also serve as further amounts of seed crystals. The crystals enriched in enantiomer pair X produced during the process can be separated and recovered from the crystallization by such methods as filtration, centrifugation or decantation of the mother liquor and the like. The choice of the separation method will in part depend on whether the crystals are to be epimerized in the same vessel in which they were formed or transferred to another vessel as appropriate in a batch, continuous or semi-continuous process.

The epimerization is suitably conducted by dissolving the enantiomer pair X in a suitable solvent as defined above and adding the desired amount of epimerization catalyst to the solution. The process may be conducted at any temperature at which epimerization proceeds without significant decomposition of the phenylacetate. The epimerization is much faster at higher temperatures. Suitably, epimerization is conducted at temperatures in the range of from −50°C to the reflux temperature of the solvent, preferably from −20°C to 150°C, and especially from 0°C to 50°C.

The product of the epimerization is essentially a solution of racemic phenylacetate. This product can then be combined with fresh quantities of racemic phenylacetate solution or with the Y-rich mother liquor

and this resulting mixture again subjected to precipitation (crystallization) under the conditions previously described above.

Neutralization or removal of the residual epimerization catalyst is carried out by known methods. In some cases, catalysts which are insoluble in the solvents of the process are advantageous, for example, basic ion exchange resin catalyst, while in other cases one may add a small amount of acidic material, for examle, acetic acid, when utilizing a basic catalyst such as ammonia.

As mentioned earlier, the mother liquor enriched in enantiomer pair Y is separated from the enantiomer pair X or X-enriched crystals by known methods, for example, by centrifuging off the X or X-enriched crystals. The enantiomer pair Y can then be concentrated or separated from the solvent, for example, by flashing off a lighter solvent such as methanol. Other light end impurities can be removed, for example, in a wiped film evaporator.

It will be appreciated by those of skill in the art that the present process can be conducted as a batch, semi-continuous or continuous process employing one or more treatment vessels as appropriate.

In its most preferred embodiment the present invention provides a process for preparing the enantiomer pair consisting of S-alpha-cyano-3-phenoxybenzyl S-alpha-isopropyl-p-chlorophenylacetate and the corresponding R-alpha-cyano-3-phenoxybenzyl R-alpha-isopropyl-p-chlorophenylacetate (hereinafter referred to as fenvalerate Y), which process comprises precipitating crystals of an enantiomer pair consisting of S-alpha-cyano-3-phenoxybenzyl R-alpha-isopropyl-p-chlorophenylacetate and R-alpha-cyano-3-phenoxybenzyl S-alpha-isopropyl-p-chlorophenylacetate (hereinafter referred to as fenvalerate X) in the presence of crystals of fenvalerate X from a solution of R,S-alpha-cyano-3-phenoxybenzyl R,S-alpha-isopropyl-p-chlorophenylacetate, separating the fenvalerate X crystals from the mother liquor, redissolving the fenvalerate X crystals in a solvent, treating the solution of fenvalerate X with a base to epimerize fenvalerate X to the corresponding racemic fenvalerate, recycling the racemic mixture back to the precipitation step and recovering fenvalerate Y from the mother liquor.

While the various catalysts, reaction conditions and solvents previously described above can be employed to prepare fenvalerate Y, it is preferable to employ as the solvent an alkanol containing from 1 to 4 carbon atoms and as the catalyst ammonia or a tertiary alkylamine containing 1 to 4 carbon atoms in each alkyl group. The use of methanol with triethylamine or especially with ammonia is preferred.

Fenvalerate X crystals have a melting point of 68°C, some 20° higher than crystals which had previously been obtained for fenvalerate Y.

The invention is illustrated by the following Examples, in which the identity of the products, including intermediates, was confirmed by gas chromatography (GC) and nuclear magnetic resonance (NMR) spectral analyses as necessary.

## Example 1

A) Preparation of crystalline fenvalerate X

A solution containing 240 g of essentially racemic technical fenvalerate 4.49 g of potassium acetate (an optional nucleating agent), 0.6 g of water and 520 g of methanol was cooled to below 0°C. Crystals began to grow rapidly, and soon there was a thick cake of crystals at the bottom of the container with supernatant solution above the cake. This mixture was allowed to stand at room temperature for several hours. Then the mixture was shaken to break up chunks of solid and a small portion was filtered. The recovered white solid, after two rinses with ice-cold methanol, was analyzed by GC as 74.2% fenvalerate X. A portion of this solid was recrystallized once from methanol to give 81.3% fenvalerate X.

The filtrate from the first filtration was chilled to 0°C and more crystals were isolated. These crystals were recrystallized from methanol acidified with acetic acid seven times, each time analyzing the product by GC. The normalized analysis of the final product was 3.7 g of fenvalerate X of 99.8% purity and melting point of 68°C.

B) Preparation of Y-rich fenvalerate

A solution containing 400 g of technical racemic fenvalerate (fenvalerate Y to fenvalerate X ratio of 47.1 to 52.9), 405 g of methanol and 0.2 g of acetic acid were combined and warmed to form a homogeneous solution. The mixture was cooled and stirred at 23°C, and 5.0 g of finely-powdered fenvalerate X crystals (prepared as in I(A) above) was added. The temperature of the slurry was slowly lowered to 0°C during about 5 hours, and a 45.9 g sample of the slurry was withdrawn and stored in the refrigerator for seeding later in the experiment. The remainder of the slurry was allowed to settle, and 360.7 g of the mother liquor was drawn off via a glass filter-stick. The bed of residual crystals was rinsed twice with portions of cold methanol (total 124.1 g) and also drawn off through the filter-stick. A total of 152.6 g was recovered. A GC analysis of the filtrate showed that the fenvalerate Y to fenvalerate X ratio had been increased to 77% to 23%, respectively.

The 359.6 g filtrate from the above crystallization was charged to a 500 ml flask and batch distilled through a 1-inch, 10-tray Oldershaw column to a kettle temperature of 105°C to recover methanol for recycle. A total of 237g of distillate was recovered and the crude fenvalerate Y-rich product remaining weighed 106 g, GC analysis indicated that the fenvalerate Y to fenvalerate X ratio in the bottoms product was 79% to 21%, respectively.

To 368 g of the wet crystals remaining after removal of the filtrate and washings from the previous

crystallization was added 140 g of methanol recovered from the distillation. The mixture was warmed to 50°C to dissolve the solids and was held at that temperature, and 0.375 ml of concentrated aqueous ammonia was added in three portions over 137 minutes to catalyze the epimerization. After a total of 250 minutes the reaction mixture was quenched by the addition of 0.265 ml of acetic acid. The fenvalerate Y to fenvalerate X ratio of the mixture was increased from an initial 36% to 64% to a ratio of better than 44% to 56% (based on sample taken after 205 minutes).

The 494 g reaction mixture from the above epimerization was cooled to room temperature, and 106 g of make-up racemic fenvalerate was added, along with the 151 g of methanolic crystal washes from the earlier crystallization and 149 g of make-up methanol from the distillation. This 765g mixture was homogenized and placed in a bath at 25°C. The 45.2 g of retained seed slurry from the previous crystallization was added, and this mixture was stirred and cooled to 0°C during 3 hours. The mixture was held at that temperature for an additional 15 hours, whereupon it was worked up in the manner described earlier. A total of 337 g of filtrate was recovered, which GC analysis showed had a fenvalerate Y to fenvalerate X ratio of 85% to 15%, respectively.

This filtrate was distilled, as before, to give 83.6 g of bottoms, which GC analysis showed had a fenvalerate Y to fenvalerate X ratio of 77% to 23%, respectively.

Examples II—V

Following procedures similar to Example I, Y-rich forms of the following phenylacetates were prepared by crystallisation of the corresponding X enantiomer pair, epimerisation and recycling:— alpha-cyano-3-phenoxybenzyl alpha-isopropyl-p-(difluoromethoxy)phenylacetate (II), alpha-cyano-3-phenoxybenzyl alpha-isopropyl-p-methylphenylacetate (III), alpha-cyano-3-phenoxybenzyl alpha-isopropyl-p-fluorophenylacetate (IV) and alpha-cyano-3-phenoxybenzyl alpha-isopropyl-p(tert-butyl)phenylacetate (V).

**Claims for the contracting states: BE LU SE**

1. A method for the preparation of a cyanobenzyl isopropylphenylacetate of the formula I:—

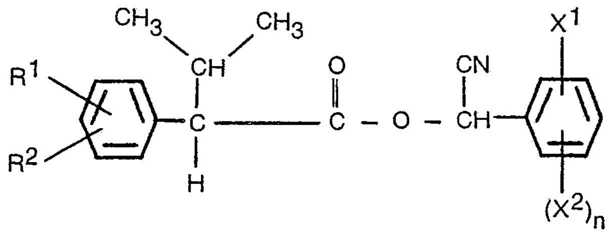

(I)

wherein $R^1$ is a hydrogen atom, a halogen atom or an alkyl group containing from 1 to 4 carbon atoms or a methoxy or ethoxy group, each optionally substituted by one or more halogen atoms; $R^2$ is a hydrogen atom or a methyl group; $X^1$ is phenoxy, benzyl or phenylthio; $X^2$ is a halogen atom, or is methyl and n is 0, 1 or 2; which is rich in S-alpha-cyanobenzyl S-alpha-isopropylphenylacetate and its enantiomer (hereinafter referred to as enantiomer pair Y), characterised in that the method comprises precipitating crystals enriched in S-alpha-cyanobenzyl R-alpha-isopropylphenylacetate and its enantiomer (hereinafter referred to as enantiomer pair X) in the presence of crystals of enantiomer pair X from a solution of the corresponding racemic R,S-alpha-cyanobenzyl R,S-alpha-isopropylphenylacetate, separating these X-enriched crystals from the mother liquor, redissolving the crystals thus separated in a solvent and treating the resulting solution with a base to epimerize the dissolved enantiomer pair X to the corresponding racemic mixture, recycling the racemic mixture back to the precipitation step and recovering mother liquor enriched in enantiomer pair Y.

2. A method as claimed in claim 1, wherein the solvent is an alkanol containing from 1 to 4 carbon atoms.

3. A method as claimed in claim 1 or 2, wherein the base is ammonia or an alkyl, aralkyl or aryl amine in which each alkyl group contains from 1 to 10 carbon atoms and any aralkyl or aryl group contains from 6 to 20 carbon atoms and 1 to 2 hydrocarbyl rings.

4. A method as claimed in claim 3, wherein the base is ammonia or triethylamine and the solvent is methanol.

5. A method as claimed in any one of claims 1—4, wherein the precipitation of crystals enriched in enantiomer pair X is conducted with the addition of seed crystals of enantiomer pair X.

6. A method as claimed in claim 5, wherein the seed crystals are added in an amount of from 0.05 to 10% based on the phenylacetate in solution.

7. A method as claimed in any one of claims 1—6 wherein an acid stabiliser is added to the reacemic phenylacetate mother liquor solution to prevent epimerisation of the enantiomer pair Y contained therein.

8. A method as claimed in claim 7, wherein the acid stabiliser is an alkanoic acid containing 1—4 carbon atoms.

9. A method as claimed in any one of claims 1—8, wherein in the compound of formula I $R^1$ is a halogen atom or an optionally halogenated alkyl or alkoxy group, $R^2$ is a hydrogen atom, $X^1$ is a 3-phenoxy substituent and n is 0.

10. A method as claimed in claim 9, wherein the cyanobenzyl phenylacetate of formula I is alpha-cyano-3-phenoxybenzyl alpha-isopropyl-p-chlorophenylacetate.

11. A pair of enantiomers of a cyanobenzyl isopropylphenylacetate of the formula I as defined in claim 1, characterised in that it comprises a crystalline enantiomer pair X, substantially free of other stereoisomers.

12. A pair of enantiomers as claimed in claim 11, which comprises alpha-cyano-3-phenoxybenzyl alpha-isopropyl-p-chlorophenylacetate in a crystalline form which contains the enantiomer pair X thereof in an amount greater than 60% by weight.

13. A pair of enantiomers as claimed in claim 12, wherein the enantiomer pair X is present in the crystalline material in an amount greater than 90% by weight.

**Claims for the contracting state: AT**

1. A method for the preparation of a cyanobenzyl isopropylphenylacetate of the formula I:—

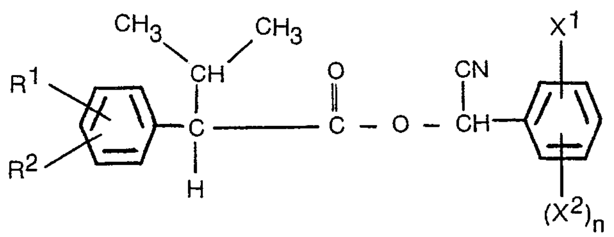
(I)

wherein $R^1$ is a hydrogen atom, a halogen atom or an alkyl group containing from 1 to 4 carbon atoms or a methoxy or ethoxy group, each optionally substituted by one or more halogen atoms; $R^2$ is a hydrogen atom or a methyl group; $X^1$ is phenoxy, benzyl or phenylthio; $X^2$ is a halogen atom, or is methyl and n is 0, 1 or 2; which is rich in S-alpha-cyanobenzyl S-alpha-isopropylphenylacetate and its enantiomer (hereinafter referred to as enantiomer pair Y), characterised in that the method comprises precipitating crystals enriched in S-alpha-cyanobenzyl R-alpha-isopropylphenylacetate and its enantiomer (hereinafter referred to as enantiomer pair X) in the presence of crystals of enantiomer pair X from a solution of the corresponding racemic R,S-alpha-cyanobenzyl R,S-alpha-isopropylphenylacetate, separating these X-enriched crystals from the mother liquor, redissolving the crystals thus separated in a solvent and treating the resulting solution with a base to epimerize the dissolved enantiomer pair X to the corresponding racemic mixture, recycling the racemic mixture back to the precipitation step and recovering mother liquor enriched in enantiomer pair Y.

2. A method as claimed in claim 1, wherein the solvent is an alkanol containing from 1 to 4 carbon atoms.

3. A method as claimed in claim 1 or 2, wherein the base is ammonia or an alkyl, aralkyl or aryl amine in which each alkyl group contains from 1 to 10 carbon atoms and any aralkyl or aryl group contains from 6 to 20 carbon atoms and 1 to 2 hydrocarbyl rings.

4. A method as claimed in claim 3, wherein the base is ammonia or triethylamine and the solvent is methanol.

5. A method as claimed in any one of claims 1—4, wherein the precipitation of crystals enriched in enantiomer pair X is conducted with the addition of seed crystals of enantiomer pair X.

6. A method as claimed in claim 5, wherein the seed crystals are added in an amount of from 0.05 to 10% based on the phenylacetate in solution.

7. A method as claimed in any one of claims 1—6 wherein an acid stabiliser is added to the reacemic phenylacetate mother liquor solution to prevent epimerisation of the enantiomer pair Y contained therein.

8. A method as claimed in claim 7, wherein the acid stabiliser is an alkanoic acid containing 1—4 carbon atoms.

9. A method as claimed in any one of claims 1—8, wherein in the compound of formula I $R^1$ is a halogen atom or an optionally halogenated alkyl or alkoxy group, $R^2$ is a hydrogen atom, $X^1$ is a 3-phenoxy substituent and n is 0.

10. A method as claimed in claim 9, wherein the cyanobenzyl phenylacetate of formula I is alpha-cyano-3-phenoxybenzyl alpha-isopropyl-p-chlorophenylacetate.

**Claims for the contracting states: CH DE FR GB IT LI NL**

1. A method for the preparation of a cyanobenzyl isopropylphenylacetate of the formula I:—

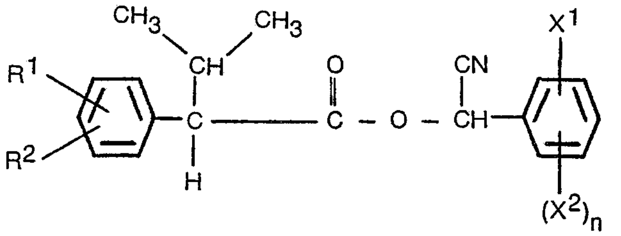

(I)

wherein $R^1$ is a hydrogen atom, a halogen atom, or an alkyl group containing from 1 to 4 carbon atoms or a methoxy or ethoxy group, each optionally substituted by one or more halogen atoms; $R^2$ is a hydrogen atom or a methyl group; $X^1$ is phenoxy, benzyl or phenylthio; $X^2$ is a halogen atom, or is methyl and n is 0, 1 or 2; which is rich in S-alpha-cyanobenzyl S-alpha-isopropylphenylacetate and its enantiomer (hereinafter referred to as enantiomer pair Y); which method comprises precipitating crystals enriched in S-alpha-cyanobenzyl R-alpha-isopropylphenylacetate and its enantiomer (hereinafter referred to as enantiomer pair X) in the presence of crystals of enantiomer pair X from a solution of the corresponding racemic R,S-alpha-cyanobenzyl R,S-alpha-isopropyl-phenylacetate, separating these X-enriched crystals from the mother liquor, redissolving the crystals thus separated in a solvent and treating the resulting solution with a base to epimerize the dissolved enantiomer pair X to the corresponding racemic mixture, recycling the racemic mixture back to the precipitation step and recovering mother liquor enriched in enantiomer pair Y, characterised in that an acid stabiliser is added to the racemic phenylacetate mother liquor to prevent epimerisation of the enantiomer pair Y contained therein.

2. A method as claimed in claim 1, wherein the acid stabiliser is an alkanoic acid containing 1 to 4 carbon atoms.

3. A method as claimed in either claim 1 or claim 2, wherein the precipitation of a crystals enriched in enantiomer pair X is conducted with the addition of seed crystals of enantiomer pair X in an amount of from 0.05 to 10% based on the phenylacetate in solution.

**Patentansprüche für die Vertragsstaaten: BE LU SE**

1. Ein Verfahren zur Herstellung eines Cyanobenzylphenylacetats der Formel I:

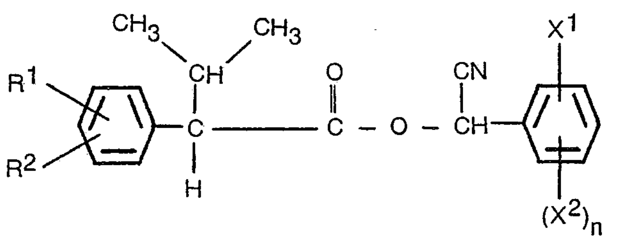

(I)

worin $R^1$ ein Wasserstoffatom, ein Halogenatom oder eine Alkylgruppe mit von 1 bis 4 Kohlenstoffatomen oder eine Methoxy- oder Ethoxygruppe darstellt, jeweils gewünschtenfalls substituiert durch ein oder mehrere Halogenatome; $R^2$ ein Wasserstoffatom oder eine Methylgruppe bedeutet; $X^1$ für Phenoxy, Benzyl oder Phenylthio steht; $X^2$ ein Halogenatom ist oder eine Methylgruppe bedeutet und n den Wert Null, 1 oder 2 hat; welches reich an S-alpha-Cyanobenzyl-S-alphaisopropylphenylacetat und dessen Enantiomer ist (nachfolgend als Enantiomerenpaar Y bezeichnet), dadurch gekennzeichnet, daß das Verfahren ein Ausfällen von an S-alpha-Cyanobenzyl-R-alpha-isopropylphenylacetat und dessen Enantiomer (in der Folge als Enantiomerenpaar X bezeichnet) angereicherten Kristallen in Gegenwart von Kristallen des Enantiomerenpaares X aus einer Lösung des entsprechenden racemischen R,S-alpha-Cyanobenzyl-R,S-alpha-isopropylphenylacetats, Abtrennen dieser X-angereicherten Kristalle von der Mutterlauge, Wiederauflösen der solcherart abgetrennten Kristalle in einem Lösungsmittel und Behandeln der gebildeten Lösung mit einer Base zum Epimerisieren des gelösten Enantiomerenpaares X zu dem entsprechenden racemischen Gemisch, Rückführen des racemischen Gemisches zur Fällungsstufe und Gewinnen von an Enantiomerenpaar Y angereicherter Mutterlauge umfaßt.

2. Ein Verfahren nach Anspruch 1, worin das Lösungsmittel ein Alkanol mit von 1 bis 4 Kohlenstoffatomen ist.

3. Ein Verfahren nach Anspruch 1 oder 2, worin die Base Ammoniak oder ein Alkyl-, Aralkyl- oder Arylamin ist, worin jede Alkylgruppe von 1 bis 10 Kohlenstoffatomen enthält und eine etwaige Aralkyl- oder Arylgruppe von 6 bis 20 Kohlenstoffatome und 1 bis 2 Hydrocarbylringe aufweist.

4. Ein Verfahren nach Anspruch 3, worin die Base Ammoniak oder Triethylamin ist und das Lösungsmittel Methanol ist.

5. Ein Verfahren nach einem der Ansprüche 1 bis 4, worin die Ausfällung der an dem Enantiomerenpaar X angereicherten Kristalle unter Zugabe von Saatkristallen des Enantiomerenpaares X vorgenommen wird.

6. Ein Verfahren nach Anspruch 5, worin die Saatkristalle in einer Menge von 0,05 bis 10%, bezogen auf das in Lösung befindliche Phenylacetat, zugesetzt werden.

7. Ein Verfahren nach einem der Ansprüche 1 bis 6, worin zu der racemischen Phenylacetat-Mutterlauge zur Verhinderung einer Epimerisation des darin enthaltenen Enantiomerenpaares Y ein saurer Stabilisator zugesetzt wird.

8. Ein Verfahren nach Anspruch 7, worin der saure Stabilisator eine 1 bis 4 Kohlenstoffatome enthaltende Alkansäure ist.

9. Ein Verfahren nach einem der Ansprüche 1 bis 8, worin in der Verbindung der Formel (I) $R^1$ ein Halogenatom oder eine gegebenenfalls halogenierte Alkyl- oder Alkoxygruppe darstellt, $R^2$ ein Wasserstoffatom bedeutet, $X^1$ einen 3-Phenoxy-Substituenten bedeutet und n den Wert Null hat.

10. Ein Verfahren nach Anspruch 9, worin das Cyanobenzylphenylacetat der Formel (I) alpha-Cyano-3-phenoxybenzylalpha-isopropyl-p-chlorphenylacetat ist.

11. Ein Enantiomerenpaar eines Cyanobenzylisopropylphenylacetats der Formel (I), wie in Anspruch 1 definiert, dadurch gekennzeichnet, daß es ein kristallines Enantiomerenpaar X, im wesentlichen frei von anderen Stereoisomeren, umfaßt.

12. Ein Enantiomerenpaar nach Anspruch 11, welches alpha-Cyano-3-phenoxybenzyl-alpha-isopropyl-p-chlorphenylacetat in einer kristallinen Form umfaßt, welche das Enantiomerenpaar X hievon in einem Anteil von über 60 Gew.-% enthält.

13. Ein Enantiomerenpaar nach Anspruch 12, worin das Enantiomerenpaar X in dem kristallinen Material in einem Anteil von über 90 Gew.-% vorliegt.

**Patentansprüche für den Vertragsstaat: AT**

1. Ein Verfahren zur Herstellung eines Cyanobenzylphenylacetats der Formel I:

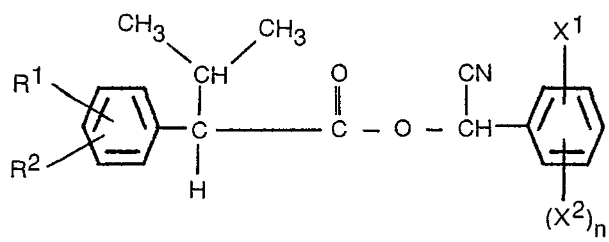

(I)

worin $R^1$ ein Wasserstoffatom, ein Halogenatom oder eine Alkylgruppe mit von 1 bis 4 Kohlenstoffatomen oder eine Methoxy- oder Ethoxygruppe darstellt, jeweils gewünschtenfalls substituiert durch ein oder mehrere Halogenatome; $R^2$ ein Wasserstoffatom oder eine Methylgruppe bedeutet; $X^1$ für Phenoxy, Benzyl oder Phenylthio steht; $X^2$ ein Halogenatom ist oder eine Methylgruppe bedeutet und n den Wert Null, 1 oder 2 hat; welches reich an S-alpha-Cyanobenzyl-S-alphaisopropylphenylacetat und dessen Enantiomer ist (nachfolgend als Enantiomerenpaar Y bezeichnet), dadurch gekennzeichnet, daß das Verfahren ein Ausfällen von an S-alpha-Cyanobenzyl-R-alpha-isopropylphenylacetat und dessen Enantiomer (in der Folge als Enantiomerenpaar X bezeichnet) angereicherten Kristallen in Gegenwart von Kristallen des Enantiomerenpaares X aus einer Lösung des entsprechenden racemischen R,S-alpha-Cyanobenzyl-R,S-alpha-isopropylphenylacetats, Abtrennen dieser X-angereicherten Kristalle von der Mutterlauge, Wiederauflösen der solcherart abgetrennten Kristalle in einem Lösungsmittel und Behandeln der gebildeten Lösung mit einer Base zum Epimerisieren des gelösten Enantiomerenpaares X zu dem entsprechenden racemischen Gemisch, Rückführen des racemischen Gemisches zur Fällungsstufe und Gewinnen von an Enantiomerenpaar Y angereicherter Mutterlauge umfaßt.

2. Ein Verfahren nach Anspruch 1, worin das Lösungsmittel ein Alkanol mit von 1 bis 4 Kohlenstoffatomen ist.

3. Ein Verfahren nach Anspruch 1 oder 2, worin die Base Ammoniak oder ein Alkyl-, Aralkyl- oder Arylamin ist, worin jede Alkylgruppe von 1 bis 10 Kohlenstoffatomen enthält und eine etwaige Aralkyl- oder Arylgruppe von 6 bis 20 Kohlenstoffatome und 1 bis 2 Hydrocarbylringe aufweist.

4. Ein Verfahren nach Anspruch 3, worin die Base Ammoniak oder Triethylamin ist und das Lösungsmittel Methanol ist.

5. Ein Verfahren nach einem der Ansprüche 1 bis 4, worin die Ausfällung der an dem Enantiomerenpaar X angereicherten Kristalle unter Zugabe von Saatkristallen des Enantiomerenpaars X vorgenommen wird.

6. Ein Verfahren nach Anspruch 5, worin die Saatkristalle in einer Menge von 0,05 bis 10%, bezogen auf das in Lösung befindliche Phenylacetat, zugesetzt werden.

7. Ein Verfahren nach einem der Ansprüche 1 bis 6, worin zu der racemischen Phenylacetat-Mutterlauge zur Verhinderung einer Epimerisation des darin enthaltenen Enantiomerenpaares Y ein saurer Stabilisator zugesetzt wird.

8. Ein Verfahren nach Anspruch 7, worin der saure Stabilisator eine 1 bis 4 Kohlenstoffatome enthaltende Alkansäure ist.

9. Ein Verfahren nach einem der Ansprüche 1 bis 8, worin in der Verbindung der Formel (I) $R^1$ ein Halogenatom oder eine gegebenenfalls halogenierte Alkyl- oder Alkoxygruppe darstellt, $R^2$ ein Wasserstoffatom bedeutet, $X^1$ einen 3-Phenoxy-Substituenten bedeutet und n den Wert Null hat.

10. Ein Verfahren nach Anspruch 9, worin das Cyanobenzylphenylacetat der Formel (I) alpha-Cyano-3-phenoxybenzylalpha-isopropyl-p-chlorphenylacetat ist.

**Patentansprüche für die Vertragsstaaten: CH DE FR GB IT NL LI**

1. Ein Verfahren zur Herstellung eines Cyanobenzylphenylacetats der Formel I:

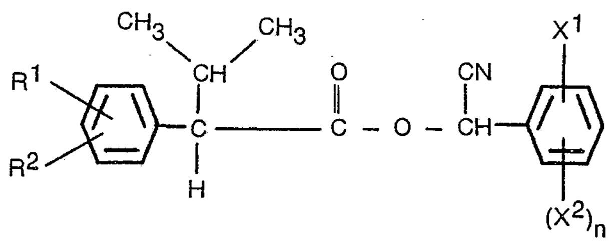

(I)

worin $R^1$ ein Wasserstoffatom, ein Halogenatom oder eine Alkylgruppe mit von 1 bis 4 Kohlenstoffatomen oder eine Methoxy- oder Ethoxygruppe darstellt, jeweils gewünschtenfalls substituiert durch ein oder mehrere Halogenatome; $R^2$ ein Wasserstoffatom oder eine Methylgruppe bedeutet; $X^1$ für Phenoxy, Benzyl oder Phenylthio steht; $X^2$ ein Halogenatom ist oder eine Methylgruppe bedeutet und n den Wert Null, 1 oder 2 hat; welches reich an S-alpha-Cyanobenzyl-S-alphaisopropylphenylacetat und dessen Enantiomer ist (nachfolgend als Enantiomerenpaar Y bezeichnet), welches Verfahren ein Ausfällen von an S-alpha-Cyanobenzyl-R-alpha-isopropylphenylacetat und dessen Enantiomer (in der Folge als Enantiomerenpaar X bezeichnet) angereicherten Kristallen in Gegenwart von Kristallen des Enantiomerenpaares X aus einer Lösung des entsprechenden racemischen R,S-alpha-Cyanobenzyl-R,S-alpha-isopropylphenylacetats, Abtrennen dieser X-angereicherten Kristalle von der Mutterlauge, Wiederauflösen der solcherart abgetrennten Kristalle in einem Lösungsmittel und Behandeln der gebildeten Lösung mit einer Base zum Epimerisieren des gelösten Enantiomerenpaares X zu dem entsprechenden racemischen Gemisch, Rückführen des racemischen Gemisches zur Fällungsstufe und Gewinnen von an Enantiomerenpaar Y angereicherter Mutterlauge umfaßt, dadurch gekennzeichnet, daß zu der racemischen Phenylacetat-Mutterlauge zur Verhinderung einer Epimerisation des darin enthaltenen Enantiomerenpaars Y ein saurer Stabilisator zugesetzt wird.

2. Ein Verfahren nach Anspruch 1, worin der saure Stabilisator eine 1 bis 4 Kohlenstoffatome aufweisende Alkansäure ist.

3. Ein Verfahren nach Anspruch 1 oder 2, worin die Ausfällung der an dem Enantiomerenpaar X angereicherten Kristalle unter Zusatz von Saatkristallen Des Enantiomerenpaares X in einer Menge von 0,05 bis 10%, bezogen auf in der Lösung vorliegendes Phenylacetat, ausgeführt wird.

**Revendications pour les Etats contractants: BE LU SE**

1. Procédé de préparation d'un cyanobenzyl-isopropylphénylacétate de formule I:

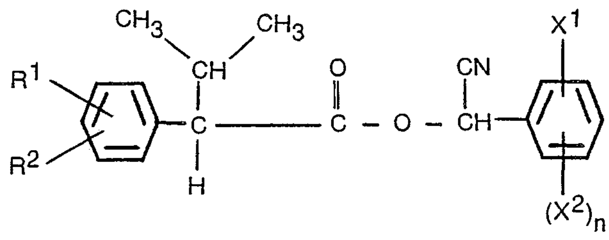

(I)

où $R^1$ est un atome d'hydrogène, un atome d'halogène ou un groupe alcoyle en $C_1$ à $C_4$ ou un groupe

méthoxy ou éthoxy, chacun éventuellement substitué par un ou plusieurs atomes d'halogène; $R^2$ est un atome d'hydrogène ou un groupe méthyle; $X^1$ est un phénoxy, un benzyle ou un phénylthio; $X^2$ est un atome d'halogène, ou est un méthyle et n vaut 0, 1 ou 2; qui est riche en S-alpha-cyanobenzyl-S-alpha-isopropylphénylacetate et son énantiomères (appelés ci-dessous paire d'enantiomeres Y), caractérisé en ce que le procédé implique de précipiter des cristaux enrichis en S-alphacyanobenzyl-R-alpha-isopropylphénylacétate et son énantiomère (appelés ci-dessous paire d'énantiomères X) en présence de cristaux de la paire d'énantiomeres X à partir d'une solution du R,S-alpha-cyanobenzyl-R,S-alpha-isopropylphénylacétate racémique correspondant, de séparer ces cristaux enrichis en X de la liqueur-mère, de redissoudre les cristaux ainsi sépares dans un solvant et de traiter la solution obtenue avec une base pour épimériser la paire d'enantiomères dissoute X en le mélange racémique correspondant, de recycler le mélange racémique vers l'étape de précipitation et de recueillir la liqueur-mère enrichie en la paire énantiomère Y.

2. Procédé selon la revendication 1, où le solvant est un alcanol en $C_1$ à $C_4$.

3. Procédé selon la revendications 1 et 2, où la base est l'ammoniac ou un alcoyle, un aralcoyle ou une arylamine où chaque groupe alcoyle contient de 1 à 10 atomes de carbone et tout groupe aralcoyle ou aryle contient de 6 à 20 atomes de carbone et de 1 à 2 noyaux hydrocarbyle.

4. Procédé selon la revendication 3, où la base est l'ammoniac ou la triéthylamine et le solvant est le méthanol.

5. Procédé selon l'une quelconque des revendications 1—4, où la précipitation des cristaux enrichis en la paire d'énantiomère? X est conduite avec addition de cristaux d'ensemencement de la paire d'énantiomères X.

6. Procédé selon la revendication 5, où les cristaux d'ensemencement sont ajoutés en une quantité allant de 0,05 à 10% sur la base du phénylacétate en solution.

7. Procédé selon l'une quelconque des revendications 1—6 où l'on ajoute un stabilisateur acide a la solution de liqueur-mère de phénylacétate racémique pour empêcher l'épimérisation de la paire d'énantiomères Y qui y est contenue.

8. Procédé selon la revendication 7, où le stabilisateur acide est un acide alcanoïque en $C_1$ à $C_4$.

9. Procédé selon l'une quelconque des revendications 1—8, où dans le composé de formule I $R^1$ est un atome d'halogène ou un groupe alcoyle ou alcoxy éventuellement halogéné, $R^2$ est un atome d'hydrogène, $X^1$ est un substituant 3-phénoxy et n vaut 0.

10. Procédé selon la revendication 9, où le cyanobenzylphénylacétate de formule I est l'alpha-cyano-3-phénoxybenzyl-alpha-isopropyl-p-chlorophénylacétate.

11. Paire d'énantiomères d'un cyanobenzyl-isopropylphénylacétate de formule I telle que féfinié dans la revendication 1, caractérisée en ce qu'elle comprend une paire d'énantiomères cristalline x, substantiellement dépourvue d'autres stéréoisomères.

12. Paire d'énantiomères selon la revendication 11, qui comprend l'alpha-cyano-3-phénoxybenzyl-alpha-isopropyl-p-chlorophénylacétate sous une forme cristalline qui contient sa paire d'énantiomères X en une quantité supérieure à 60% en poids.

13. Paire d'énantiomères selon la revendication 12, où la paire d'enantiomères X est présente dans la matière cristalline en une quantité supérieure à 90% en poids.

**Revendications pour l'Etat contractant: AT**

1. Procédé de préparation d'un cyanobenzyl-isopropylphénylacétate de formule I:

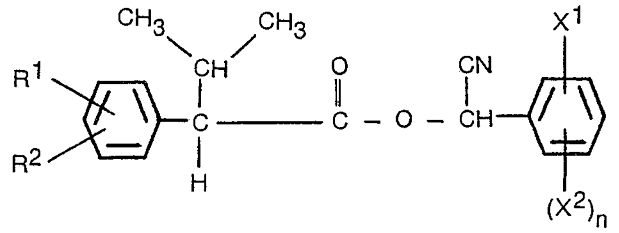

(I)

où $R^1$ est un atome d'hydrogène, un atome d'halogène ou un groupe alcoyle en $C_1$ à $C_4$ ou un groupe méthoxy ou éthoxy, chacun éventuellement substitué par un ou plusieurs atomes d'halogène; $R^2$ est un atome d'hydrogène ou un groupe méthyle; $X^1$ est un phénoxy, un benzyle ou un phénylthio; $X^2$ est un atome d'halogène, ou est un méthyle et n vaut 0, 1 ou 2; qui est riche en S-alpha-cyanobenzyl-S-alpha-isopropylphénylacetate et son énantiomères (appelés ci-dessous paire d'enantiomeres Y), caractérisé en ce que le procédé implique de précipiter des cristaux enrichis en S-alphacyanobenzyl-R-alpha-isopropylphénylacétate et son énantiomère (appelés ci-dessous paire d'énantiomères X) en présence de cristaux de la paire d'énantiomeres X à partir d'une solution du R,S-alpha-cyanobenzyl-R,S-alpha-isopropylphénylacétate racémique correspondant, de séparer ces cristaux enrichis en X de la liqueur-mère, de redissoudre les cristaux ainsi sépares dans un solvant et de traiter la solution obtenue avec une base pour épimériser la

paire d'enantiomères dissoute X en le mélange racémique correspondant, de recycler le mélange racémique vers l'étape de précipitation et de recueillir la liqueur-mère enrichie en la paire énantiomère Y.

2. Procédé selon la revendication 1, où le solvant est un alcanol en $C_1$ à $C_4$.

3. Procédé selon la revendications 1 et 2, où la base est l'ammoniac ou un alcoyle, un aralcoyle ou une arylamine où chaque groupe alcoyle contient de 1 à 10 atomes de carbone et tout groupe aralcoyle ou aryle contient de 6 à 20 atomes de carbone et de 1 à 2 noyaux hydrocarbyle.

4. Procédé selon la revendication 3, où la base est l'ammoniac ou la triéthylamine et le solvant est le méthanol.

5. Procédé selon l'une quelconque des revendications 1—4, où la précipitation des cristaux enrichis en la paire d'énantiomères X est conduite avec addition de cristaux d'ensemencement de la paire d'énantiomères X.

6. Procédé selon la revendication 5, où les cristaux d'ensemencement sont ajoutés en une quantité allant de 0,05 à 10% sur la base du phénylacétate en solution.

7. Procédé selon l'une quelconque des revendications 1—6 où l'on ajoute un stabilisateur acide a la solution de liqueur-mère de phénylacétate racémique pour empêcher l'épimérisation de la paire d'énantiomères Y qui y est contenue.

8. Procédé selon la revendication 7, où le stabilisateur acide est un acide alcanoïque en $C_1$ à $C_4$.

9. Procédé selon l'une quelconque des revendications 1—8, où dans le composé de formule I $R^1$ est un atome d'halogène ou un groupe alcoyle ou alcoxy éventuellement halogéné, $R^2$ est un atome d'hydrogène, $X^1$ est un substituant 3-phénoxy et n vaut 0.

10. Procédé selon la revendication 9, où le cyanobenzylphénylacétate de formule I est l'alpha-cyano-3-phénoxybenzyl-alpha-isopropyl-p-chlorophénylacétate.

**Revendications pour les Etats contractants: CH DE FR GB IT NL LI**

1. Procédé de préparation d'un cyanobenzyl-isopropylphénylacétate de formule I:

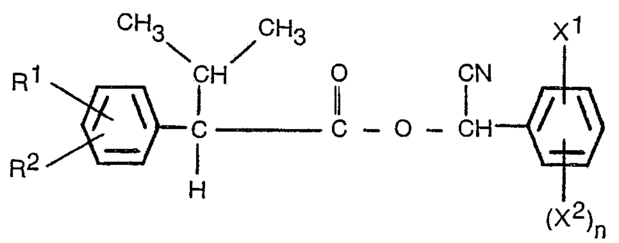

(I)

où $R^1$ est un atome d'hydrogène, un atome d'halogène ou un groupe alcoyle en $C_1$ à $C_4$ ou un groupe méthoxy ou éthoxy, chacun éventuellement substitué par un ou plusieurs atomes d'halogène; $R^2$ est un atome d'hydrogène ou un groupe méthyle; $X^1$ est un phénoxy, un benzyle ou un phénylthio; $X^2$ est un atome d'halogène, ou est un méthyle et n vaut 0, 1 ou 2; qui est riche en S-alpha-cyanobenzyl-S-alpha-isopropylphénylacetate et son énantiomère (appelés ci-dessous paire d'enantiomeres Y); lequel procédé implique de précipiter des cristaux enrichis en S-alpha-cyanobenzyl-R-alpha-isopropylphénylacétate et son énantiomère (appelés ci-dessous paire d'énantiomères X) en présence de cristaux de la paire d'énantiomeres X provenant d'une solution du R,S-alpha-cyanobenzyl-R,S-alpha-isopropyl-phénylacétate racémique correspondant, de séparer ces cristaux enrichis en X de la liqueur-mère, de redissoudre les cristaux ainsi sépares dans un solvant et de traiter la solution obtenue avec une base pour épimériser la paire d'enantiomères dissoute X en le mélange racémique correspondant, de recycler le mélange recémique vers l'étape de précipitation et de recueillir la liqueurmère enrichie en paire d'énantiomères Y, caractérisé en ce que l'on ajoute un stabilisateur acide à la liqueurmère phénylacétate racémique pour empêcher l'épimérisation de la paire d'énantiomères Y qui y est contenue.

2. Procédé selon la revendication 1, où le stabilisateur acide est un acide alcanoique en $C_1$ à $C_4$.

3. Procédé selon l'une des revendications 1 et 2, où la précipitation de cristaux enrichis en la paire d'énantiomères X est conduite avec addition de cristaux d'ensemencement de la paire d'énantiomères X en une quantité allant de 0,05 à 10% sur la base du phénylacétate en solution.